# EUROPÄISCHE PATENTANMELDUNG

(11) **EP 0 748 796 A2**
(43) Veröffentlichungstag der Anmeldung: **18.12.1996**
(21) Anmeldenummer: 96106790.7
(22) Anmeldetag: 30.04.1996
(51) Int. Cl.: C07C 309/12, C07C 303/32, C11D 1/12

(54) **Verfahren zur Herstellung von fliessfähigen, wasserfreien Acyloxyalkansulfonaten und deren Verwendung**

(30) Priorität: 09.05.1995 DE 19516865
(71) Anmelder: HOECHST AKTIENGESELLSCHAFT, 65929 Frankfurt am Main (DE)
(72) Erfinder: Tittel, Viliam, 65719 Hofheim (DE); Bühring, Dirk, Dr., Jardim Natal, 08613-010 Suzano - SP (BR)

(57) **Zusammenfassung**

Beim erfindungsgemäßen Verfahren wird im Rahmen der Veresterung von Fettsäuren mit Hydroxyalkansulfonaten das 180 bis 250 °C heiße Reaktionsprodukt (Schmelzmasse) während des Abkühlens auf 150 bis 50 °C so geschert, daß die mittlere Teilchengröße des Produktes < 100 µm ist. Das so erhaltene Acyloxyalkansulfonat-Produkt ist auch noch bei Temperaturen im Bereich von 60 bis 100 °C gut fließbar, pumpbar und dergleichen und bei diesen Temperaturen auch über eine lange Zeitdauer in flüssigem Zustand ohne Veränderung lagerbar.

## Beschreibung

Die Erfindung betrifft ein Verfahren zur Herstellung von fließfähigen, wasserfreien Acyloxyalkansulfonaten durch Veresterung von Fettsäuren mit Hydroxyalkansulfonaten und deren Verwendung zur Herstellung von Seifen.

Acyloxyalkansulfonate sind wertvolle anionische Tenside, die vor allem zur Bereitung von Syndet-Seifen, kosmetischen Mitteln und Reinigungsformulierungen eingesetzt werden. Ihre Herstellung erfolgt vorteilhaft durch Veresterung von mindestens einer Fettsäure mit mindestens einem Hydroxyalkansulfonat (Direktveresterung). Ein solches Verfahren ist zum Beispiel in EP-A-0 585 071 (US-A-5 384 421) beschrieben. Dabei werden die Fettsäure und das Salz der Hydroxyalkansulfonsäure in Gegenwart eines Veresterungskatalysators bei einer Temperatur von 180 bis 240 °C umgesetzt unter gleichzeitiger Entfernung von vorhandenem Wasser. Das Reaktionsprodukt (in Form einer Schmelze) ist bei Temperaturen bis herab auf etwa 150 °C flüssig. Um ein Produkt zu erhalten, das auch noch bei einer relativ niedrigen Temperatur fließfähig ist, wird das Reaktionsprodukt zunächst mit Fettsäure versetzt und auf einen pH-Wert von 6 bis 7 eingestellt. Die neutrale Mischung wird dann mit Wasser in einer Menge von 25 bis 90 Gew.-% kombiniert. Das als wäßrige Lösung oder Dispersion erhaltene Acyloxyalkansulfonat ist nun bis herab auf etwa 50 °C pumpbar und kann auch bei etwa 50 °C in flüssiger Form gelagert werden.

Pumpbare Produkte von Acyloxyalkansulfonaten werden auch in WO-A-93/16155 beschrieben. Sie bestehen im wesentlichen aus 20 bis 60 % Acyloxyalkansulfonat, 2 bis 50 % Paraffin, 20 bis 55 % Wasser, 0 bis 7 % Hydroxyalkansulfonat und gegebenenfalls 5 bis 25 % Fettsäure. Sie werden in der Weise hergestellt, daß man a) das Paraffin und gegebenenfalls die Fettsäure auf oder über deren Schmelzpunkt erhitzt, b) das Acyloxyalkansulfonat, das Wasser und gegebenenfalls das Hydroxyalkansulfonat zur Mischung a) dazugibt und c) die Komposition auf eine Temperatur von 38 bis 71 °C abkühlt, wobei die Komposition mit einer hohen Scherrate andauernd gerührt wird bis die Partikelgröße weniger als 50 µm beträgt. Das so erhaltene Produkt mit 20 bis 55 % Wasser ist bis herab auf etwa 50 °C flüssig und pumpbar.

Acyloxyalkansulfonat-Produkte, die gut fließen, das heißt gut fließbar, gießbar, pumpbar und dergleichen sind, besitzen den Vorteil einer besseren Handhabung. Der Einsatz von Wasser dazu bringt aber mehrere Nachteile mit sich. So führt das Wasser zur Erhöhung der Transportkosten, da es keinen Wertstoff darstellt. Bei mehreren Weiterverarbeitungen der wäßrigen Acyloxyalkansulfonat-Lösungen zu Fertigprodukten muß das Wasser wieder entfernt werden, zum Beispiel bei der Bereitung von Syndet-Seifen. Das Wasser kann auch dazu führen, daß das Acyloxyalkansulfonat hydrolysiert.

Die Aufgabe der Erfindung besteht demnach darin, ein Verfahren vorzuschlagen, mit dem ohne Einsatz von Wasser oder anderen Lösungsmitteln Acyloxyalkansulfonat-Produkte mit einem hohen Gehalt an Aktivsubstanz erhalten werden, die bei relativ niedrigen Temperaturen gut fließen und in flüssigem Zustand auch lagerbar und lagerstabil sind, unter anderem auch hinsichtlich Hydrolyse und Phasentrennung.

Das erfindungsgemäße Verfahren zur Herstellung von fließfähigen, wasserfreien Acyloxyalkansulfonaten durch Veresterung von Fettsäuren mit Hydroxyalkansulfonaten ist dadurch gekennzeichnet, daß man mindestens eine Fettsäure der Formel 1 RCOOH (1), worin R ein Kohlenwasserstoffrest mit 5 bis 31 Kohlenstoffatomen ist, mit mindestens einem Hydroxyalkansulfonat der Formel 2 HO-R¹-SO₃X (2), worin R¹ ein C₂ bis C₄-Alkylen oder ein divalenter Di-C₂ bis C₄-alkyletherrest ist und X ein Alkalimetall oder Ammonium ist, in Gegenwart eines Veresterungskatalysators bei einer Temperatur von 180 bis 250 °C, vorzugsweise 190 bis 230 °C, unter Entfernen des anwesenden Wassers umsetzt, das Reaktionsprodukt auf eine Temperatur von 150 bis 50 °C, vorzugsweise 110 bis 60 °C, abkühlt und während des Abkühlens so schert, daß die mittlere Teilchengröße des Produktes weniger als 100 µm beträgt.

Acyloxyalkansulfonat kristallisiert schon bei höheren Temperaturen in Form von nadelförmigen Kristallen, wodurch eine in sich verfilzte und besonders feste Masse anfällt. Die Erfindung beruht auf der überraschenden Feststellung, daß dies dann verhindert werden kann und ein auch bei niedrigeren Temperaturen noch fließfähiges und pumpbares Produkt ohne Einsatz von Wasser oder anderen Lösungs- oder Dispergiermitteln erhalten wird, wenn man während des erwähnten Abkühlens die Struktur der Kristallmasse zerschlägt und die nadelförmigen Kristalle zerkleinert und in ihrer Morphologie verändert. Das Produkt, das nun eine mittlere Teilchengröße von < 100 µm aufweisen soll, ist auch noch bei Temperaturen im Bereich von etwa 60 bis 100 °C gut gießbar, pumpbar und dergleichen und bei dieser Temperatur auch über eine lange Zeitdauer in flüssigem Zustand ohne Veränderung lagerbar.

Beim erfindungsgemäßen Verfahren wird das 180 bis 250 °C, vorzugsweise 190 bis 230 °C, heiße Reaktionsprodukt nach Erreichen des angestrebten Umsetzungsgrades auf eine Temperatur von 150 bis 50 °C abgekühlt, vorzugsweise 110 bis 60 °C, zum Beispiel durch kontinuierliches Abkühlen des Reaktionsgefäßes (Mantelkühlung). Während der gesamten Abkühlphase, die im allgemeinen 2 bis 6 Stunden dauert, wird die Masse solchen Scherkräften unterworfen, daß ihre mittlere Teilchengröße nach Erreichen der genannten Temperatur im Bereich von 150 bis 50 °C, vorzugsweise 110 bis 60 °C, bei unter 100 µm liegt, vorzugsweise bei etwa 10 bis 80 µm. Die geforderte hohe Scherung wird zum Beispiel mit Misch-, Rühr- oder Homogenisiereinrichtungen wie Dispergierscheiben, Homogenisatoren, Cavitron-Geräten, Rotor-Stator-Mischern und dergleichen erreicht. In der Reaktionsphase, das heißt von Beginn der Umsetzung bis zum gewünschten Umsetzungsgrad und der Einleitung der Abkühlphase, genügt die übliche Bewegung der Schmelze, zum Beispiel mit einem normalen Ankerrührer. Um einen Tausch eines üblichen Rührers mit einer Schereinrichtung zu vermeiden, kann es zweckmäßig sein, die Schereinrichtung schon zu Beginn der Reaktion einzusetzen. Entscheidend ist, daß die genannte Scherung in der Abkühlphase vorgenommen wird. Das so erhaltene Acyloxyalkansulfonat-Produkt weist bei Temperaturen bis herab auf etwa 100 bis 60 °C die angegebenen Eigenschaften bezüglich Fließfähigkeit auf.

Die erfindungsgemäße Umsetzung von Fettsäure und Hydroxyalkansulfonat wird in Gegenwart eines Katalysators durchgeführt. Geeignete Veresterungskatalysatoren sind in der genannten EP-A-0 585 071 ausführlich beschrieben, die hier miteinbezogen wird. Es handelt sich um Alkansulfonsäuren, Hydroxyalkansulfonsäuren, Arylsulfonsäuren, anorganischen Säuren wie Schwefelsäure, Phosphorsäure, phosphorige Säure, Borsäure oder deren Anhydride, Schwermetallsalze wie Zinksulfat, Zirkoniumsulfat, Zinkisethionat, Zinkborat, Aluminiumsulfat, Titansulfat oder Wolframphosphat, Metalloxide wie Zinkoxid, Aluminiumoxid, Magnesiumoxid, Ceroxid, Zirkoniumoxid oder Lanthanoxid, ferner um Mischungen von zwei oder mehreren der genannten Katalysatoren und um Seifen, die aus Schwermetallen und Metalloxiden gebildet werden. Ein besonders bevorzugter Veresterungskatalysator ist Zinkoxid. Der Veresterungskatalysator wird in einer Menge von im allgemeinen 0,05 bis 2 Gew.-%, vorzugsweise 0,05 bis 1 Gew.-%, eingesetzt, Gewichtsprozente bezogen auf Fettsäure und Hydroxyalkansulfonsäuresalz.

Die Umsetzung von Fettsäure und Hydroxyalkansulfonsäuresalz, im allgemeinen im Molverhältnis von 1 : 1 bis 2 : 1, vorzugsweise etwa 1 : 1, wird erfindungsgemäß bei einer Temperatur von 180 bis 250 °C durchgeführt. Der bevorzugte Temperaturbereich liegt bei 190 bis 230 °C. Das gegebenenfalls mit den Ausgangskomponenten in die Reaktionsmischung eingebrachte Wasser und das durch die Veresterungsreaktion entstehende Wasser wird kontinuierlich aus der Reaktionsmischung ausgetragen. Die Reaktionsmischung kann unter Umständen mehr oder weniger hochviskos werden. In solchen Fällen wird man sogenannte Konsistenzregler einsetzen, zum Beispiel spezielle Fettsäuren und/oder Paraffine, wie sie in EP-A-0 585 071 beschrieben sind. Die Zeit bis zum angestrebten Umsatz liegt bei etwa 4 bis 8 Stunden. Im allgemeinen wird man bei einem Umsatz von etwa 50 bis 80 Gew.-% Acyloxyalkansulfonat (Aktivsubstanz) mit dem Abkühlen der Schmelze und Abbrechen der Veresterungsreaktion beginnen.

Das erfindungsgemäße Verfahren kann im einzelnen zum Beispiel in der Weise durchgeführt werden, daß man - bei Atmosphärendruck - die Fettsäure, das Salz der Hydroxyalkansulfonsäure und den Veresterungskatalysator in einem Reaktionsgefäß vorlegt und die Mischung unter Rühren auf die angegebene Temperatur erhitzt. Vorhandenes Wasser destilliert bereits während des Erhitzens der Reaktionsmischung und dann weiter während der laufenden Veresterungsreaktion kontinuierlich ab. Das erfindungsgemäße Verfahren kann auch nach der in EP-A-0 585 071 beschriebenen Methode durchgeführt werden. Hier wird die Veresterungsreaktion teils bei Atmosphärendruck und teils unter Anwendung eines Vakuums zur schnelleren Austragung des Wassers durchgeführt. Nach Erreichen des angestrebten Umsetzungsgrades wird das Produkt wie oben beschrieben abgekühlt und unter Einwirkung von Scherkräften behandelt. Es weist nun die genannte Teilchengröße auf und ist auch noch im Bereich von 60 bis 100 °C gut fließend und flüssig lagerbar. Bei weiterer Abkühlung wird das Produkt fest. Eine Konfektionierung des festen Produktes kann man zum Beispiel mit Hilfe einer Schuppenwalze oder eines Kühlbandes durchführen.

Zu den Ausgangsverbindungen Fettsäure und Hydroxyalkansulfonat sei noch folgendes gesagt: In der Fettsäure RCOOH ist R ein Kohlenwasserstoffrest mit 5 bis 31 Kohlenstoffatomen, der gesättigt oder ungesättigt, gerade oder verzweigt sein kann. R kann auch ein Gemisch von solchen Kohlenwasserstoffresten sein. R ist bevorzugt C₅ bis C₂₁-Alkyl oder C₅ bis C₂₁-Alkenyl oder eine Mischung davon. Die Alkenylreste sind vorzugsweise ein- bis dreifach ungesättigt. Als Beispiele seien genannt Capronsäure, Caprinsäure, Laurinsäure, Myristinsäure, Stearinsäure, Arachinsäure, Ölsäure, Linolsäure, Linolensäure, Cocosfettsäure und Talgfettsäure sowie Mischungen davon. Der verzweigte Kohlenwasserstoffrest R ist bevorzugt ein Rest der nachstehenden Formel 1a 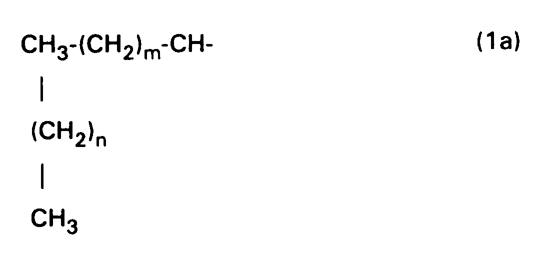 worin m eine ganze Zahl von 4 bis 18 ist, vorzugsweise 4 bis 14, und n 0 oder eine ganze Zahl von 1 bis 10 ist, vorzugsweise 0 oder eine ganze Zahl von 1 bis 6. Solche Fettsäuren entsprechen also der nachstehenden Formel 1b 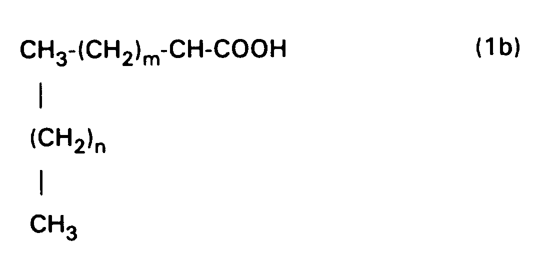 worin m und n die angegebenen Bedeutungen haben. Beispiele für verzweigte Fettsäuren sind: 2-Ethylhexansäure, 2-Pentyloctansäure, 2-Butylnonansäure, 2-Propyldecansäure, 2-Ethylundecansäure, 2-Butylundecansäure, 2-Methyldodecansäure, 2-Ethyltridecansäure und 2-Methyltetradecansäure und Mischungen davon.

Als Hydroxyalkansulfonate HO-R¹-SO₃X sind solche bevorzugt, worin R¹ -CH₂CH₂-, -(CH₂)₃-, -CH₂CH(CH₃)- oder -CH₂CH₂OCH₂CH₂- ist, wobei Ethylen besonders bevorzugt ist, und das Gegenion X NH₄ oder ein Alkalimetall, vorzugsweise Natrium oder Kalium, ist. Das erfindungsgemäß besonders bevorzugte Salz von Hydroxyalkansulfonsäuren ist Kalium-, Natrium- oder Ammoniumisethionat. Die Hydroxyalkansulfonsäuresalze können als solche eingesetzt werden, bevorzugt werden sie in Form einer wäßrigen Lösung, im allgemeinen als 40 bis 65gew. %ige Lösung, eingesetzt.

Die erfindungsgemäß hergestellten wasserfreien Acyloxyalkansulfonat-Produkte, die zum Beispiel in beheizten Straßentankzügen ohne weiteres transportiert werden können, werden bevorzugt zur Bereitung von Syndet-Seifen verwendet. Sie eignen sich dazu deshalb in besonderer Weise, weil sie als solche weiterverarbeitet werden können (sie enthalten kein Wasser oder andere Lösungsmittel und Fremdkomponenten, die vorher entfernt werden müßten) und weil sie aufgrund ihrer feinen Teilchengröße Seifen mit einer besonders glatten Oberfläche ergeben.

Das erfindungsgemäße Verfahren ist auch großtechnisch durchführbar. Durch die Direktveresterung (Direktkondensation) kann auf den Einsatz von Fettsäurechloriden verzichtet werden, die sonst in einem gesonderten Schritt aus Fettsäure hergestellt werden müßten. Die erfindungsgemäß erhaltenen Acyloxyalkansulfonat-Produkte weisen als weitere vorteilhafte Eigenschaften - neben den oben beschriebenen - eine gute Wasserlöslichkeit und Hartwasserstabilität, starke Schaumbildung und gute Hautverträglichkeit auf. Die erfindungsgemäß hergestellten wasserfreien Schmelzen enthalten, wie oben erwähnt, im allgemeinen 50 bis 80 Gew.-% Acyloxyalkansulfonat (Aktiv- oder Wirksubstanz), bezogen auf das feste oder flüssige Gesamtprodukt. Das Acyloxyalkansulfonat entspricht der nachstehenden Formel 3 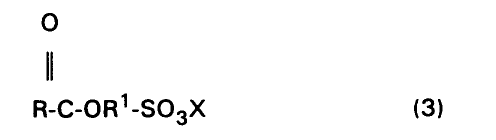 worin R, R¹ und X die angegebenen Bedeutungen haben.

Die Erfindung wird nun an Beispielen (erfindungsgemäß) und Vergleichsbeispielen (nicht erfindungsgemäß) noch näher erläutert. Prozentangaben beziehen sich auf das Gewicht, wenn keine anderen Angaben gemacht sind.

### Vergleichsbeispiel 1

In einem 3-l-Planschliffbecher mit Ankerrührer, absteigender Destillationsbrücke, Innenthermometer und Stickstoffeinleitung werden 876 g Cocosfettsäure, 776 g wäßrige Natriumisethionat-Lösung (57 %) und 2,4 g Zinkoxid vorgelegt. Die Mischung wird auf 220 °C erwärmt und das Lösewasser des Natriumisethionats sowie das bei der Direktkondensation gebildete Wasser abdestilliert. Bei einem Natriumcocoylisethionat-Gehalt von 75 % werden 147 g Paraffin, 178 g Stearinsäure, 78 g Laurinsäure und 78 g Polyethylenglykol 2000 zugegeben und die Schmelzmasse unter Rühren auf 67 °C abgekühlt. Sie wird dann bei 80 °C gelagert. Nach 2 Tagen Lagerzeit ist das Produkt noch fließfähig. Nun wird es auf 150 °C erwärmt und diesmal ohne Rührung auf 80 °C abgekühlt, wobei es nach kurzer Zeit fest wird.

### Vergleichsbeispiel 2

In einem 3-l-Planschliffbecher mit Ankerrührer, absteigender Destillationsbrücke, Innenthermometer und Stickstoffeinleitung werden 876 g Cocosfettsäure, 776 g wäßrige Natriumisethionat-Lösung (57 %) und 2,2 g Zinkoxid vorgelegt. Der Ansatz wird auf 230 °C erwärmt und das Lösewasser des Natriumisethionats sowie das bei der Direktkondensation gebildete Wasser abdestilliert. Bei einem Natriumcocoylisethionat-Gehalt von 78 % werden 750 g Paraffin und 140 g Stearinsäure zugegeben und die Mischung unter Rühren auf 80 °C abgekühlt und bei dieser Temperatur gelagert. Nach 3 Tagen Lagerzeit ist das Produkt pastös und läßt sich nicht vollständig aus dem Planschliffbecher ausgießen.

### Beispiel 1

Durchführung wie im Vergleichsbeispiel 2, wobei anstelle des Ankerrührers eine Dispergierscheibe eingesetzt wird. Im Gegensatz zu Vergleichsbeispiel 2 ist das Produkt, das eine Teilchengröße von im Mittel etwa 80 µm hat, auch nach 14 Tagen Lagerzeit bei 80 °C dünnflüssig und läßt sich vollständig aus dem Planschliffbecher ausgießen.

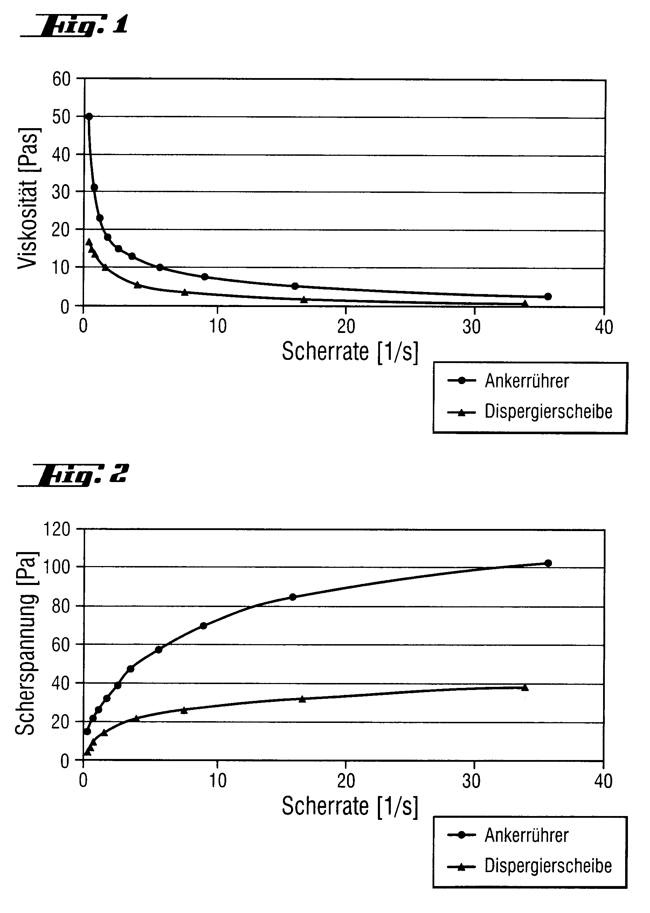

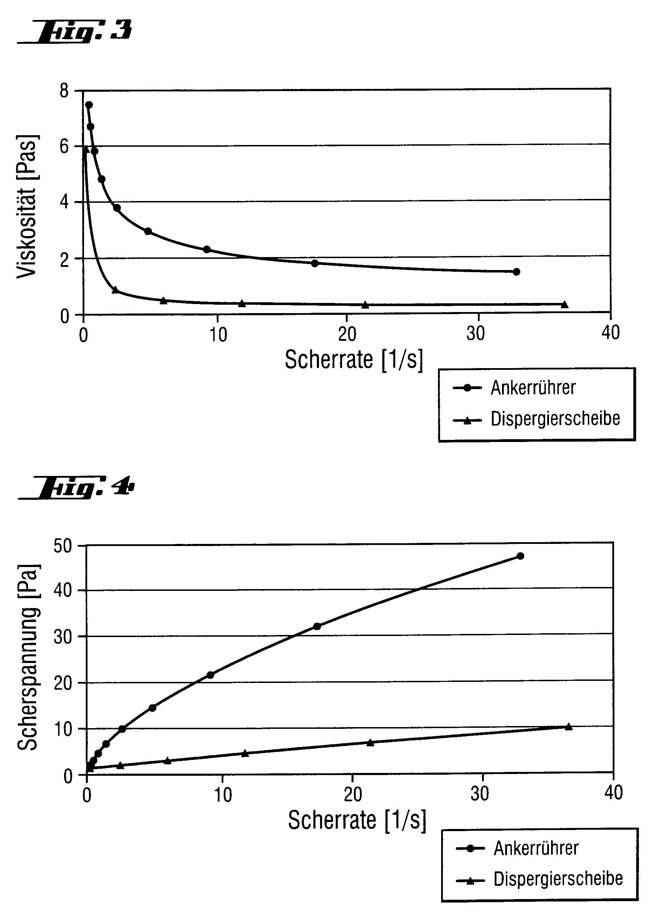

Die Abbildungen 1 und 2 zeigen die Fließkurven der beiden Produkte.

### Vergleichsbeispiel 3

In einem 3-l-Planschliffbecher mit Ankerrührer, absteigender Destillationsbrücke, Innenthermometer und Stickstoffeinleitung werden 876 g Cocosfettsäure, 776 g wäßrige Natriumisethionat-Lösung (57 %) und 2,2 g Zinkoxid vorgelegt. Der Ansatz wird auf 230 °C erwärmt und das Lösewasser des Natriumisethionats sowie das bei der Direktkondensation gebildete Wasser abdestilliert. Bei einem Natriumcocoylisethionat-Gehalt von 78 % werden 470 g Paraffin und 210 g Stearinsäure zugegeben und die Schmelze unter Rühren auf 80 °C abgekühlt und bei dieser Temperatur gelagert. Nach 1 Tag Lagerzeit ist das Produkt pastös und läßt sich nicht aus dem Planschliffbecher ausgießen.

### Beispiel 2

Durchführung wie im Vergleichsbeispiel 3, wobei anstelle des Ankerrührers eine Dispergierscheibe eingesetzt wird. Im Gegensatz zu Vergleichsbeispiel 3 ist das Produkt, das eine mittlere Teilchengröße von etwa 50 µm hat, auch nach 14 Tagen Lagerzeit bei 60 °C dünnflüssig und läßt sich vollständig aus dem Planschliffbecher ausgießen.

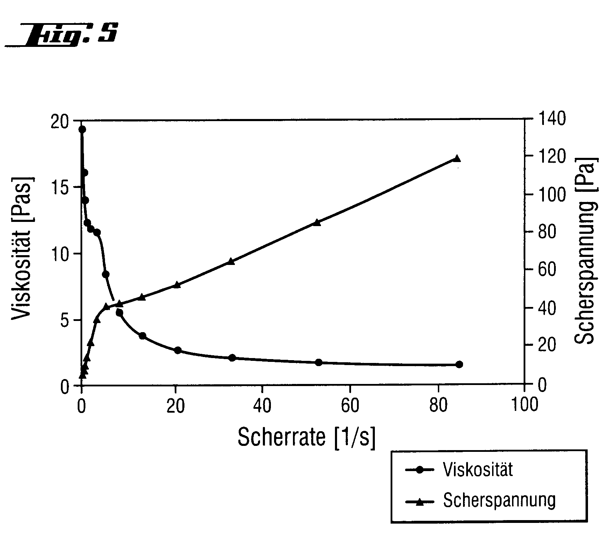

Die Abbildungen 3 und 4 zeigen die Fließkurven der beiden Produkte.

### Beispiel 3

In einem 3-l-Planschliffbecher mit einer Dispergierscheibe als Rühr- und Mischeinrichtung, absteigender Destillationsbrücke, Innenthermometer und Stickstoffeinleitung werden 533 g Cocosfettsäure, 511 g wäßrige Natriumisethionat-Lösung (57 %) und 1,6 g Zinkoxid vorgelegt. Der Ansatz wird auf 230 °C erwärmt und das Lösewasser des Natriumisethionats sowie das bei der Direktkondensation gebildete Wasser abdestilliert. Bei einem Natriumcocoylisethionat-Gehalt von 77 % werden circa 40 g der im Überschuß eingesetzten Cocosfettsäure abdestilliert und anschließend 187 g Stearinsäure zugegeben und die Schmelze unter Rühren auf 95 °C abgekühlt. Das Produkt blieb bei einer Lagertemperatur von circa 100 °C über den Zeitraum von 6 Tagen eine pumpbare Paste.

Wird das Produkt nicht mit einer Dispergierscheibe, sondern unter Ankerrührung hergestellt, so verfestigt es sich bei circa 140 °C. Die Fließkurve ist in Abbildung 5 dargestellt.

### Beispiel 4

In einem 360-l-Rührkessel mit Ankerrührer, absteigender Destillationsvorrichtung, Stickstoffeinleitung und Temperaturmessung im Kessel werden 63 kg Oxethansalzlösung, 65 kg Cocosfettsäure, 6 kg Laurinsäure und 0,2 kg Zinkoxidpulver vorgelegt und anschließend unter Destillation von Wasser auf 220 °C geheizt. Nach Beendigung der Kondensation werden 40 kg Paraffin zugegeben und die Schmelze unter Umpumpen durch ein Cavitron-Gerät auf 150 °C abgekühlt. Bei dieser Temperatur werden 14 kg Stearinsäure zugesetzt und anschließend die Schmelze bei weiterem Umpumpen durch das Cavitron-Gerät auf 80 °C abgekühlt und aus dem Kessel in Fässer abgefüllt. Das Produkt wird bei 80 °C mehrere Tage gelagert, wobei es pumpbar und fließfähig bleibt.

### Vergleichsbeispiel 4

Durchführung analog Beispiel 4. Umpumpeinrichtung mit Umwälzpumpe und Cavitron-Gerät sind während des Abkühlens nicht eingeschaltet. In diesem Fall wird das Produkt bei 80 °C schon nach wenigen Stunden fest und ist nicht mehr fließfähig.

## Patentansprüche

1. Verfahren zur Herstellung von fließfähigen, wasserfreien Acyloxyalkansulfonaten durch Veresterung von Fettsäuren mit Hydroxyalkansulfonaten, dadurch gekennzeichnet, daß man mindestens eine Fettsäure der Formel 1 RCOOH (1), worin R ein Kohlenwasserstoffrest mit 5 bis 31 Kohlenstoffatomen ist, mit mindestens einem Hydroxyalkansulfonat der Formel 2 HO-R¹-SO₃X (2), worin R¹ ein C₂ bis C₄-Alkylen oder ein divalenter Di-C₂ bis C₄-alkyletherrest und X ein Alkalimetall oder Ammonium ist, in Gegenwart eines Veresterungskatalysators bei einer Temperatur von 180 bis 250 °C unter Entfernen des anwesenden Wassers umsetzt, das Reaktionsprodukt auf eine Temperatur von 150 bis 50 °C abkühlt und während des Abkühlens so schert, daß die mittlere Teilchengröße des Produktes weniger als 100 µm beträgt.

2. Verfahren nach Anspruch 1, dadurch gekennzeichnet, daß R ein gerader oder verzweigter Alkylrest mit 5 bis 21 Kohlenstoffatomen oder ein gerader oder verzweigter Alkenylrest mit 5 bis 21 Kohlenstoffatomen oder eine Mischung davon ist, R¹ -CH₂CH₂-, -(CH₂)₃-, -CH₂CH(CH₃)- oder -CH₂CH₂OCH₂CH₂- und X Natrium, Kalium oder Ammonium ist.

3. Verfahren nach Anspruch 1 oder 2, dadurch gekennzeichnet, daß die Umsetzung bei einer Temperatur von 190 bis 230 °C durchgeführt wird.

4. Verfahren nach einem oder mehreren der Ansprüche 1 bis 3, dadurch gekennzeichnet, daß die Fettsäure und das Hydroxyalkansulfonat im Molverhältnis von 1 : 1 bis 2 : 1 eingesetzt werden.

5. Verfahren nach einem oder mehreren der Ansprüche 1 bis 4, dadurch gekennzeichnet, daß als Veresterungskatalysator Zinkoxid in einer Menge von 0,05 bis 2 Gew.-%, bezogen auf Fettsäure und Hydroxyalkansulfonat, eingesetzt wird.

6. Verfahren nach einem oder mehreren der Ansprüche 1 bis 5, dadurch gekennzeichnet, daß das Reaktionsprodukt auf eine Temperatur von 110 bis 60 °C abgekühlt wird.

7. Verfahren nach einem oder mehreren der Ansprüche 1 bis 6, dadurch gekennzeichnet, daß während des Abkühlens so geschert wird, daß die mittlere Teilchengröße des Produktes 10 bis 80 µm beträgt.

8. Verfahren nach Anspruch 1, dadurch gekennzeichnet, daß man mindestens eine Fettsäure der Formel 1, worin R ein gerader oder verzweigter Alkylrest mit 5 bis 21 Kohlenstoffatomen oder ein gerader oder verzweigter Alkenylrest mit 5 bis 21 Kohlenstoffatomen oder eine Mischung davon ist, mit mindestens einem Hydroxyalkansulfonat der Formel 2, worin R¹ -CH₂CH₂-, -(CH₂)₃-, -CH₂CH(CH₃)- oder -CH₂CH₂OCH₂CH₂- und X Natrium, Kalium oder Ammonium ist, im Molverhältnis von 1 : 1 bis 2 : 1 in Gegenwart von Zinkoxid in einer Menge von 0,05 bis 2 Gew.-%, bezogen auf Fettsäure und Hydroxyalkansulfonat, bei einer Temperatur von 180 bis 250 °C umsetzt, das Reaktionsprodukt auf eine Temperatur von 150 bis 50 °C abkühlt und während des Abkühlens so schert, daß die mittlere Teilchengröße des Produktes weniger als 100 µm beträgt.

9. Verfahren nach Anspruch 8, dadurch gekennzeichnet, daß bei einer Temperatur von 190 bis 230 °C umgesetzt, auf eine Temperatur von 110 bis 60 °C abgekühlt und während des Abkühlens so geschert wird, daß die mittlere Teilchengröße des Produktes 10 bis 80 µm beträgt.

10. Verwendung der Acyloxyalkansulfonate nach den Ansprüchen 1 bis 9 zur Bereitung von Syndet-Seifen.
